# EUROPEAN PATENT APPLICATION

(11) **EP 4 732 773 A1**
(43) Date of publication of application: **29.04.2026**
(21) Application number: 24851863.1
(22) Date of filing: 06.08.2024
(51) Int. Cl.: A61B 5/332, A61B 5/256, A61B 5/346, A61B 5/352, A61B 5/353

(54) **BIOSIGNAL MEASUREMENT DEVICE**

(30) Priority: 09.08.2023 JP 2023130331
(71) Applicant: OMRON Corporation, Kyoto-shi, Kyoto 600-8530 (JP); Omron Healthcare Co., Ltd., Muko-shi, Kyoto 617-0002 (JP)
(72) Inventor: WANG, Danni, Kyoto-shi, Kyoto 600-8530 (JP); KOIZUMI, Masayuki, Kyoto-shi, Kyoto 600-8530 (JP); KIMURA ISHIDA, Yui, Kyoto-shi, Kyoto 600-8530 (JP); KUBO, Mitsuaki, Kyoto-shi, Kyoto 600-8530 (JP); KAWABATA, Yasuhiro, Muko-shi, Kyoto 617-0002 (JP); FUJII, Kenji, Muko-shi, Kyoto 617-0002 (JP)
(74) Representative: Isarpatent
(86) International application number: PCT/JP2024/028128
(87) International publication number: WO 2025/033440

(57) **Abstract**

To provide a biosignal measurement device that can achieve low power consumption while achieving highly accurate electrocardiographic measurement. The biosignal measurement device includes: a plurality of electrodes; a measurement unit including a detection circuit that detects a potential difference using the plurality of electrodes and configured to measure an electrocardiographic signal of a subject using the detection circuit; a battery configured to supply power to the detection circuit; and a selection unit configured to select a measurement electrode to be used for measurement of the electrocardiographic signal of the subject from the plurality of electrodes, based on the electrocardiographic signal of the subject, which is measured using at least some of the plurality of electrodes and the detection circuit. In addition, the measurement unit measures the electrocardiographic signal using the detection circuit and the measurement electrode.

## Description

### TECHNICAL FIELD

The present invention relates to a biosignal measurement device.

### BACKGROUND OF INVENTION

A wearable type electrocardiograph is known that is worn on the body continuously for a long period of time to monitor an electrocardiographic signal. There are a variety of products ranging from devices for medical use, such as 24-hour Holter electrocardiographs, to consumer devices such as smart watches. For example, Patent Literature 1 proposes a Holter electrocardiograph that determines, based on impedance, the number of channels for recording an electrocardiogram.

### CITATION LIST

### PATENT LITERATURE

Patent Literature 1: JP 6778043 A

### SUMMARY

### TECHNICAL PROBLEM

In an electrocardiograph including a plurality of electrodes, power consumption is reduced by attaching only some of the plurality of electrodes and enabling only detection circuits corresponding to the attached electrodes. However, in the case of an electrocardiograph that is attached by a user himself/herself, such as an upper arm electrocardiograph, some of the attached electrodes are not always arranged at positions suitable for electrocardiographic measurement. Therefore, in a case where electrodes other than the some electrodes are not attached, the electrocardiographic measurement may be hindered.

An object of one aspect of the disclosed technology is to provide a biosignal measurement device that can achieve low power consumption while achieving highly accurate electrocardiographic measurement.

### SOLUTION TO PROBLEM

One aspect of the disclosed technique is exemplified by the following biosignal measurement device. The biosignal measurement device includes: a plurality of electrodes; a measurement unit including a detection circuit that detects a potential difference using the plurality of electrodes and configured to measure an electrocardiographic signal of a subject using the detection circuit; a battery configured to supply power to the detection circuit; and a selection unit configured to select a measurement electrode to be used for measurement of the electrocardiographic signal of the subject from the plurality of electrodes, based on the electrocardiographic signal of the subject, which is measured using at least some of the plurality of electrodes and the detection circuit. The measurement unit measures the electrocardiographic signal using the detection circuit and the measurement electrode.

With the biosignal measurement device, the measurement electrode is selected based on the electrocardiographic signal of the subject measured using at least some of the electrodes. Therefore, the biosignal measurement device can select the measurement electrode and the detection circuit that are suitable for electrocardiographic measurement. In addition, since the electrocardiographic signal is measured using the selected measurement electrode, the power consumption of the detection circuit is reduced as compared with a case where the electrocardiographic signal is measured using all the electrodes. Thus, the biosignal measurement device can achieve low power consumption while achieving highly accurate electrocardiographic measurement.

The biosignal measurement device may have the following feature. When an abnormality is detected in the electrocardiographic signal measured using the at least some of the plurality of electrodes, the selection unit increases the number of the electrodes to be selected as the measurement electrodes. When an abnormality is detected in the electrocardiographic signal, it is preferable that the electrocardiographic signal is analyzed in detail. With the biosignal measurement device having such a feature, when an abnormality is detected in the electrocardiographic signal, more electrodes can be used for measurement of the electrocardiographic signal by the measurement unit, and thus, an electrocardiographic signal as precise as possible can be acquired. Here, examples of the abnormality of the electrocardiographic signal can include a case where a deviation of an R-R Interval (RRI) in a time-series change of the electrocardiographic signal is equal to or greater than a first threshold value, a case where a P wave is not detected in the electrocardiographic signal, a case where a magnitude of noise included in the electrocardiographic signal is equal to or greater than a second threshold value, and the like. Note that when an abnormality is detected in the electrocardiographic signal measured using the at least some of the plurality of electrodes, the selection unit may select all the plurality of electrodes as the measurement electrodes.

The biosignal measurement device may have the following feature. The selection unit allows the measurement of the electrocardiographic signal using all the plurality of electrodes to be executed at predetermined intervals, and determines, based on the electrocardiographic signal measured using all the plurality of electrodes, which electrode of the plurality of electrodes is to be the measurement electrode. The biosignal measurement device having such a feature can compare the electrocardiographic signals measured for all the plurality of electrodes and select, as the measurement electrode, an electrode more suitable for acquiring an electrocardiographic signal.

The biosignal measurement device may have the following feature. A fixing member having a belt-like configuration to be wound on the subject is further provided, and the plurality of electrodes are disposed on the fixing member. The biosignal measurement device having such a feature is easy to attach the biosignal measurement device to the subject.

### ADVANTAGEOUS EFFECTS OF INVENTION

According to the disclosed technique, low power consumption can be achieved while highly accurate electrocardiographic measurement can be achieved.

### BRIEF DESCRIPTION OF THE DRAWINGS

[FIG. 1] FIG. 1 is a diagram illustrating a state where an electrocardiograph according to an embodiment is worn on an upper arm.
[FIG. 2] FIG. 2 is a plan view of the electrocardiograph according to the embodiment.
[FIG. 3] FIG. 3 is a perspective view of the electrocardiograph according to the embodiment.
[FIG. 4] FIG. 4 is a diagram schematically illustrating connection of electrodes, detection circuits, and a battery of the electrocardiograph according to the embodiment.
[FIG. 5] FIG. 5 is a block diagram illustrating an example of a functional configuration of a control body.
[FIG. 6] FIG. 6A to FIG. 6D are diagrams schematically illustrating selection of a supplied circuit by a selection unit.
[FIG. 7] FIG. 7 is a diagram illustrating an example of a processing flow of the control body according to the embodiment.
[FIG. 8] FIG. 8 is a first diagram illustrating a variation of the connection between the electrodes and a detection circuit in a first modified example.
[FIG. 9] FIG. 9 is a second diagram illustrating a variation of the connection between the electrodes and a detection circuit in the first modified example.
[FIG. 10] FIG. 10 is a third diagram illustrating a variation of the connection between the electrodes and detection circuits in the first modified example.
[FIG. 11] FIG. 11 is a diagram schematically illustrating selection of the electrodes used for acquiring an electrocardiographic signal in a second modified example.

### DESCRIPTION OF EMBODIMENTS

### <Example of Application>

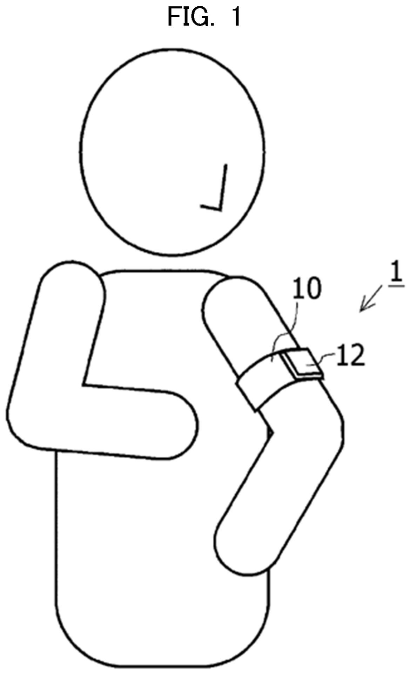

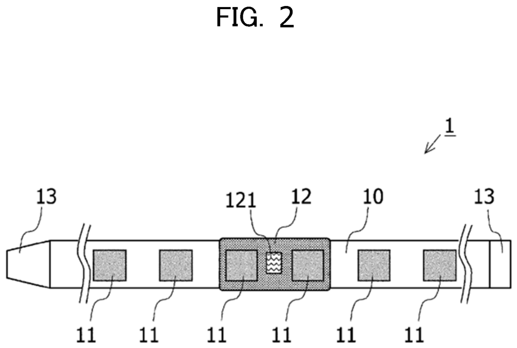

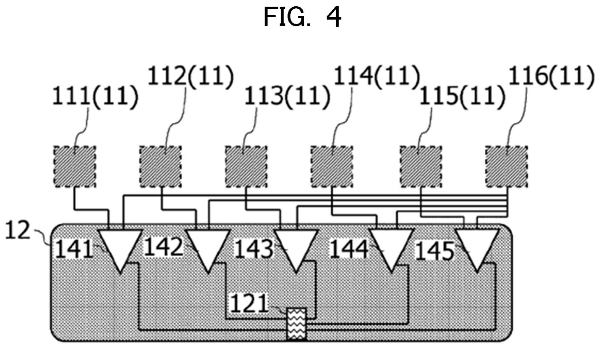

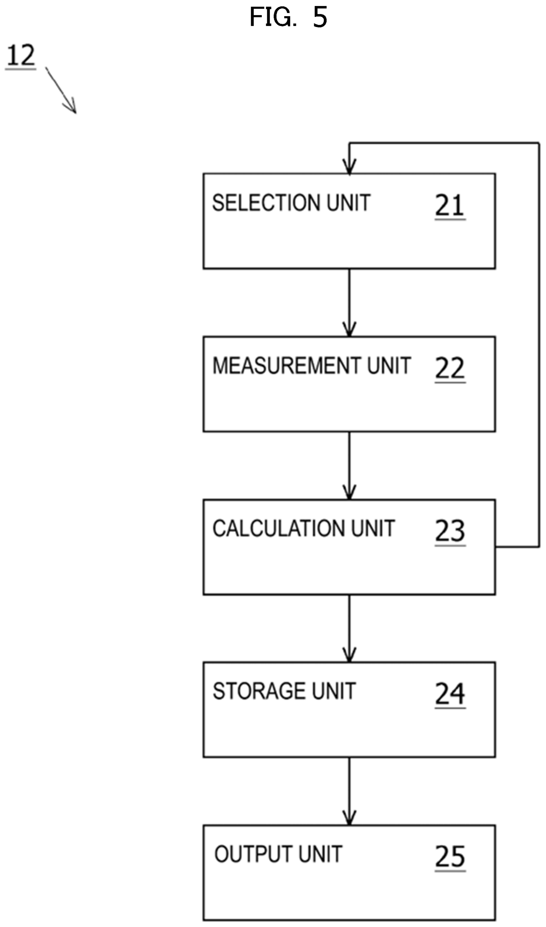

An example of application of the present invention will be described. An electrocardiograph 1 according to the present example of application is, for example, a wearable electrocardiograph that is fixed by wrapping a band 10 around a measurement site of a subject as illustrated in FIG. 1. A plurality of electrodes 11 and a control body 12 are fixed to the band 10 (see FIG. 2). The control body 12 includes detection circuits 14 (see FIG. 4) each detecting a potential difference using the plurality of electrodes 11, and includes a measurement unit 22 (see FIG. 5) that measures an electrocardiographic signal of the subject using the detection circuit 14. Examples of measurement sites where the band 10 can be attached include upper limbs (upper arms, forearms, wrists, hands, and fingers), lower limbs (thighs, lower legs, ankles, feet, and toes), head, neck, chest, abdomen, earlobe, and the like and a measurement site is appropriately selected depending on a measurement algorithm and the like.

The electrocardiographic signal used for electrocardiographic measurement is acquired using, for example, two electrodes 11 (one electrode pair). The electrocardiographic signal is, for example, a signal indicating an electrical activity of the heart. In the electrocardiograph 1, a selection unit 21 (see FIG. 5) selects measurement electrodes to be used for measuring an electrocardiographic signal from the plurality of electrodes 11 based on the electrocardiographic signal acquired from at least some of the plurality of electrodes 11. According to such an electrocardiograph 1, the measurement electrodes are selected based on the electrocardiographic signal measured using the detection circuit 14 and at least some of the electrodes 11, and thus the measurement electrodes suitable for electrocardiographic measurement can be selected. In addition, according to the electrocardiograph 1, since the number of the electrodes 11 used as the measurement electrodes can be reduced, power consumption of the detection circuit 14 can be reduced, and thus low power consumption of the electrocardiograph 1 can be achieved.

### <Embodiments>

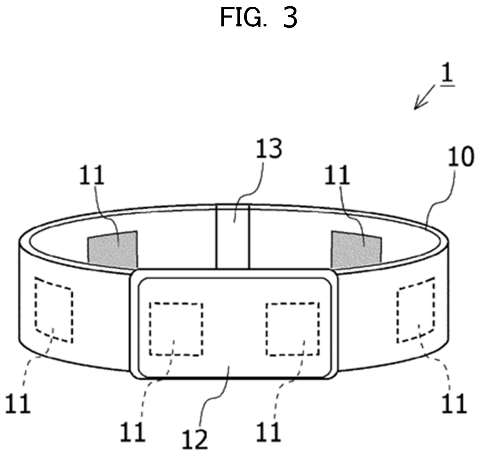

An embodiment will be described below with reference to the drawings. FIG. 1 is a diagram illustrating a state in which the electrocardiograph 1 according to an embodiment is worn on an upper arm. FIG. 2 is a plan view of the electrocardiograph 1 according to the embodiment. FIG. 3 is a perspective view of the electrocardiograph 1 according to the embodiment. The electrocardiograph 1 is not limited to a device that performs only electrocardiographic measurement, and may be a biosignal measurement device that also measures a biosignal other than electrocardiographic signals of blood pressure, a blood oxygen level, and the like.

The electrocardiograph 1 of the present embodiment is an upper arm electrocardiographic device that is worn on an upper arm (preferably, a left upper arm close to the heart) of a user and is used to measure an electrocardiographic signal as a biosignal.

The electrocardiograph 1 includes, as main components, the band 10, the plurality of electrodes 11 fixed to the band 10, and the control body 12 fixed to the band 10.

The band 10 is a member (fixing member) for fixing the electrodes 11 in a state where the electrodes 11 are pressed against a living body. In the present embodiment, a belt-shaped band 10 made of a flexible and pliable material (for example, chemical fiber, silicon, leather, or the like) is used. A fixing mechanism 13 is provided at a longitudinal end portion of the band 10. As illustrated in FIG. 1 and FIG. 3, the electrocardiograph 1 can be worn on the upper arm by forming the band 10 into a loop shape and fastening the band 10 with the fixing mechanism 13. The fixing mechanism 13 may be any mechanism such as a hook-and-loop fastener, a hook, a connector, a button, a magnet, or the like.

The plurality of electrodes 11 (also referred to as an electrode array) are embedded and fixed in the band 10 such that a contact surface with the living body is exposed to an inner side (living body side) of the band 10. The plurality of electrodes 11 are arranged in a line at equal intervals in the longitudinal direction of the band 10. Thus, when the band 10 is wrapped around the arm, the electrodes 11 come into contact with different positions on the circumference of the arm. The number of the electrodes 11 is not particularly limited and can be designed as appropriate. At least two electrodes 11 (one electrode pair) may be provided to measure an electrocardiographic signal, and three or more electrodes 11 may be provided to increase reliability and robustness of measurement. In the present embodiment, a configuration in which six electrodes 11 are provided is adopted.

The electrode 11 is a dry-type metal electrode. A wet-type electrode (gel electrode or the like) has problems such as a possibility of causing skin rash or itching when attached for a long time, and low durability and maintainability, whereas the dry-type electrode 11 does not have such problems. The electrocardiograph 1 of the present embodiment is assumed to be worn continuously for a long time and to monitor the electrocardiographic signal for 24 hours, and thus the electrodes 11 are preferably dry-type electrodes.

The control body 12 is a processing unit that performs control and signal processing of the electrocardiograph 1. The control body 12 has a structure in which a processor, a memory, a battery 121, and other circuits are mounted inside a case made of, for example, resin or metal. The control body 12 may be provided with a physical switch and a display.

FIG. 4 is a diagram schematically illustrating connection of the electrodes 11, the detection circuits 14, and the battery 121 of the electrocardiograph 1 according to the embodiment. In FIG. 4, the electrodes 11 connected to the detection circuits 14 are also illustrated by the dotted lines. When the six electrodes 11 are distinguished from each other, the electrodes 11 are referred to as electrodes 111, 112, 113, 114, 115, and 116. One electrode 116 of the six electrodes 11 is a GND electrode, a potential of which is fixed to the ground.

The control body 12 includes five detection circuits 14. When the five detection circuits 14 are distinguished from each other, the detection circuits 14 are also referred to as detection circuits 141, 142, 143, 144, and 145. The detection circuit 14 is a circuit that is used for detecting a potential difference using the connected electrode 11 by receiving power supply from the battery 121. The detection circuit 14 includes, for example, a differential amplifier. The detection circuit 141 is connected to the electrode 111 and the electrode 116, and detects a potential difference between the electrode 111 and the electrode 116. The detection circuit 142 is connected to the electrode 112 and the electrode 116, and detects a potential difference between the electrode 112 and the electrode 116. The detection circuit 143 is connected to the electrode 113 and the electrode 116, and detects a potential difference between the electrode 113 and the electrode 116. The detection circuit 144 is connected to the electrode 114 and the electrode 116, and detects a potential difference between the electrode 114 and the electrode 116. The detection circuit 145 is connected to the electrode 115 and the electrode 116, and detects a potential difference between the electrode 115 and the electrode 116. The battery 121 may be a primary battery or a secondary battery.

### <Control Body>

FIG. 5 is a block diagram illustrating an example of a functional configuration of the control body 12. The control body 12 includes the selection unit 21, the measurement unit 22, a calculation unit 23, a storage unit 24, and an output unit 25. In the control body 12, a processor executes a program stored in the storage unit 24, thereby implementing each processing unit such as the selection unit 21, the measurement unit 22, the calculation unit 23, and the output unit 25. In other words, the control body 12 can be regarded as a computer. Each processing units such as the selection unit 21, the measurement unit 22, the calculation unit 23, and the output unit 25 may be implemented by a dedicated hardware circuit.

The selection unit 21 selects, based on an electrocardiographic signal acquired by at least a part of the detection circuits 14, a supplied circuit of the detection circuits 14, which receives power supply from the battery 121. The selection unit 21 selects the supplied circuit from the detection circuits 14 based on, for example, the presence or absence of a heart disease event detected based on the electrocardiographic signal, the magnitude of noise included in the electrocardiographic signal, the amount of electrocardiographic signal to be acquired, the beat rate, the R-R Interval (RRI), and the like. Since different pairs of the electrodes 11 are connected to the detection circuits 141, 142, 143, 144, and 145, the selection unit 21 can also select the measurement electrode to be used for measurement of the electrocardiographic signal.

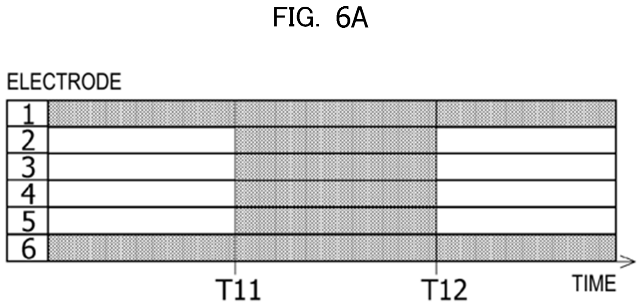

FIG. 6A to FIG. 6D are diagrams schematically illustrating selection of the supplied circuit by the selection unit 21. In FIG. 6A to FIG. 6D, unique numbers "1" to "6" are assigned to the six electrodes 11, respectively, and periods in which power is supplied from the battery 121 to the detection circuits 14 corresponding to the respective electrodes 11 are colored. The electrode 11 with the number "1" corresponds to the electrode 111, the electrode 11 with the number "2" corresponds to the electrode 112, the electrode 11 with the number "3" corresponds to the electrode 113, the electrode 11 with the number "4" corresponds to the electrode 114, the electrode 11 with the number "5" corresponds to the electrode 115, and the electrode 11 with the number "6" corresponds to the electrode 116.

In FIG. 6A, power is always supplied to the detection circuit 141 to which the electrodes 11 with "1" and "6" are connected. In other words, in FIG. 6A, the electrodes 11 with "1" and "6" are always selected as the measurement electrodes. In FIG. 6A, it is assumed that a symptom of a heart disease such as atrial fibrillation is detected at time T11 based on an electrocardiographic signal that is acquired by the measurement unit 22 using the electrodes 11 with "1" and "6" and the detection circuit 141 to which the electrodes 11 are connected. Here, an example of the symptom of the heart disease such as atrial fibrillation can include a case where a deviation of the RRI between electrocardiographic waveforms becomes equal to or greater than a predetermined threshold value. In this case, the selection unit 21 starts, at time T11, supplying power to the detection circuits 142, 143, 144, and 145 to which the electrodes 11 with "2", "3", "4", and "5" are connected. In other words, the selection unit 21 allows the measurement unit 22 to start acquisition of electrocardiographic signals by using all the electrodes 11 and the detection circuits 141, 142, 143, 144, and 145 that are included in the electrocardiograph 1. At this stage, all the electrodes with "1", "2", "3", "4", "5", and "6" are selected as the measurement electrodes. When the symptom of the heart disease is no longer detected at time T12, the selection unit 21 stops the supply of power to the detection circuits 142, 143, 144, and 145 to which the electrodes 11 with "2", "3", "4", and "5" are connected, and continues the supply of power to the detection circuit 141 to which the electrodes 11 with "1" and "6" are connected. At this stage, the electrodes 11 with "1" and "6" are selected as the measurement electrodes. In other words, in the example of FIG. 6A, the acquisition of electrocardiographic signals is started, by using the detection of the heart disease as a trigger, with all the electrodes 11 and the detection circuits 14 set as the supplied circuits, and thus the acquisition of as many electrocardiographic signals as possible is achieved while the symptom of the heart disease is detected.

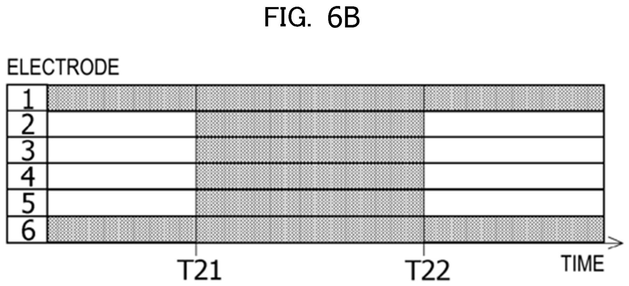

In FIG. 6B, power is always supplied to the detection circuit 141 to which the electrodes 11 with "1" and "6" are connected. In other words, in FIG. 6B, the electrodes 11 with "1" and "6" are always selected as the measurement electrodes. In FIG. 6B, it is assumed that the noise included in the electrocardiographic signal that is acquired by the measurement unit 22 using the electrodes 11 with "1" and "6" and the detection circuit 141 to which the electrodes 11 are connected becomes equal to or greater than a threshold value at time T21. In this case, the selection unit 21 starts, at time T21, supply of power to the detection circuits 142, 143, 144, and 145 to which the electrodes 11 with "2", "3", "4", and "5" are connected. In other words, the selection unit 21 allows the measurement unit 22 to start acquisition of electrocardiographic signals by using all the electrodes 11 and the detection circuits 141, 142, 143, 144, and 145 that are included in the electrocardiograph 1. At this stage, all the electrodes 11 with "1", "2", "3", "4", "5", and "6" are selected as the measurement electrodes. When the noise included in the electrocardiographic signal becomes less than the thresholds at time T22, the selection unit 21 stops the supply of power to the detection circuits 142, 143, 144, and 145 to which the electrodes 11 with "2", "3", "4", and "5" are connected, and allows the measurement unit 22 to continue acquisition of electrocardiographic signals by using the electrodes 11 with "1" and "6" and the detection circuit 141 to which the electrodes 11 are connected. At this stage, the electrodes 11 with "1" and "6" are selected as the measurement electrodes. In other words, in the example illustrated in FIG. 6B, the electrocardiographic signals are acquired by all the electrodes 11 and the detection circuits 14 while the noise included in the electrocardiographic signals is equal to or greater than the threshold value, and thus the electrocardiographic signals can be acquired from the electrodes 11, which have less noise, of the six electrodes 11.

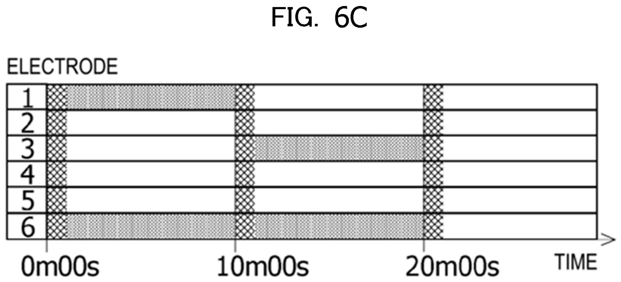

Referring to FIG. 6C, an example will be described in which power is supplied to all the detection circuits 14 connected to the electrodes 11 for a predetermined period, and the selection of the electrodes 11 to be used is performed at a predetermined cycle (one cycle of 10 minutes in the example of FIG. 6C) based on the RRI of the electrocardiographic signal acquired in the predetermined period. At the time of activation (0 minutes and 0 seconds) of the electrocardiograph 1, power is supplied to all the detection circuits 14 for a predetermined period (for example, for 30 seconds). During time from the activation of the electrocardiograph 1 to 10 minutes and 0 seconds, the selection unit 21 selects the detection circuit 14 to which power is supplied, based on the RRI of the electrocardiographic signal acquired in the period of 30 seconds. In the example of FIG. 6C, it is assumed that the detection circuit 141 to which the electrodes 11 with "1" and "6" are connected is selected. At this stage, the electrodes 11 with "1" and "6" are selected as the measurement electrodes. When 10 minutes and 0 seconds have elapsed after the activation of the electrocardiograph 1, the selection unit 21 supplies power to all the detection circuits 14 for a predetermined time, and selects, by 20 minutes and 0 seconds, the electrode 11 to which power is supplied. In the example of FIG. 6C, it is assumed that the detection circuit 143 to which the electrodes 11 with "3" and "6" are connected is selected. At this stage, the electrodes 11 with "3" and "6" are selected as the measurement electrodes.

Subsequently, when 20 minutes and 0 seconds have elapsed after the activation of the electrocardiograph 1, the selection unit 21 supplies power to all the detection circuits 14 for a predetermined time, and selects, by 30 minutes and 0 seconds, the detection circuit 14 to which power is supplied. In the example of FIG. 6C, the selection unit 21 determines, based on the electrocardiographic signal acquired during time from 30 minutes and 0 seconds to the predetermined time, to stop the supply of power to all the detection circuits 14. Examples of the case of determining to stop the supply of power to all the detection circuits 14 can include a case where an electrocardiographic signal corresponding to a predetermined beat rate (for example, 30 beats) is acquired, a case where an electrocardiographic signal corresponding to a predetermined time (for example, for 30 seconds) is acquired, and a case where it is determined based on the electrocardiographic signal that there is no heart abnormality. An example of the case where it is determined that there is no heart abnormality can include, for example, a case where the RRI is constant. As just described, the selection unit 21 may supply power to all the detection circuits 14 and repeatedly execute processing of selecting the detection circuit 14 to be used, based on the acquired electrocardiographic signal at a predetermined cycle.

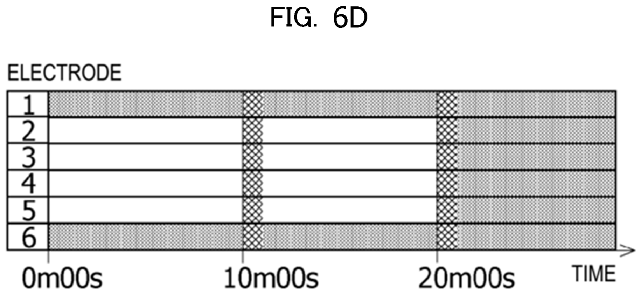

Referring to FIG. 6D, an example will be described in which power is supplied to all the detection circuits 14 for a predetermined period and it is determined based on the RRI of the electrocardiographic signal acquired in the predetermined period whether power is supplied to only the detection circuit 14 corresponding to some of the electrodes 11 in the future. At the time of activation of the electrocardiograph 1, the selection unit 21 starts supply of power to the detection circuit 141 to which some of the electrodes 11 (the electrodes 11 with "1" and "6" in FIG. 6D) are connected. At this stage, the electrodes 11 with "1" and "6" are selected as the measurement electrodes. When 10 minutes and 0 seconds have elapsed after the activation of the electrocardiograph 1, the selection unit 21 supplies power to all the detection circuits 14 for a predetermined time, and determines whether to supply power only to the detection circuit 141 to which the electrodes 11 with "1" and "6" are connected in the future. In this determination, for example, when the RRI of the electrocardiographic signal is regular, it may be determined to supply power only to the detection circuit 141 in the future. Also, in this determination, for example, when the RRI of the electrocardiographic signal is irregular, it may be determined to supply power to more detection circuits 14 (for example, all the detection circuits 14). An example of the case where the RRI is irregular can include a case where a deviation of the RRI in a plurality of waveforms is equal to or greater than a predetermined threshold in an electrocardiographic waveform that indicates a time-series change of the acquired electrocardiographic signal. In the example of FIG. 6D, at 10 minutes and 0 seconds after the activation of the electrocardiograph 1, power is supplied to all the detection circuits 14 for a predetermined time, and the selection unit 21 determines to continue the supply of power only to the detection circuit 141. At this stage, the electrodes 11 with "1" and "6" are selected as the measurement electrodes. In the example of FIG. 6D, at 20 minutes and 0 seconds after the activation of the electrocardiograph 1, power is supplied to all the detection circuits 14 for a predetermined time, and the selection unit 21 determines to continue the supply of power to all the detection circuits 14. At this stage, all the electrodes 11 with "1", "2", "3", "4", "5", and "6" are selected as the measurement electrodes.

Referring back to FIG. 5, the measurement unit 22 performs electrocardiographic measurement using the detection circuit 14 selected by the selection unit 21. The measurement unit 22 is, for example, a circuit that acquires, from the detection circuit 14, a potential difference in the electrode pair connected to the detection circuit 14 selected by the selection unit 21 and outputs the potential difference as an electrocardiographic signal. The calculation unit 23 calculates measurement results such as a beat rate and the presence or absence of atrial fibrillation by using the electrocardiographic signal. The calculation unit 23 stores the calculated measurement results in the storage unit 24. The storage unit 24 is, for example, a nonvolatile storage unit.

The output unit 25 is, for example, a liquid crystal display (LCD), a plasma display panel (PDP), an inorganic electroluminescence (EL) panel, or an organic EL panel. The output unit 25 displays, for example, information indicating the electrode 11 selected by the selection unit 21 and a time-series change (electrocardiographic waveform) of the electrocardiographic signal acquired by the measurement unit 22. For example, a touch panel that detects a touch operation by a finger or the like of the user may be provided on the output unit 25 in an overlapping manner. By providing the touch panel on the output unit 25 in an overlapping manner, the electrocardiograph 1 can offer an intuitive operation environment to the user.

### <Processing Flow>

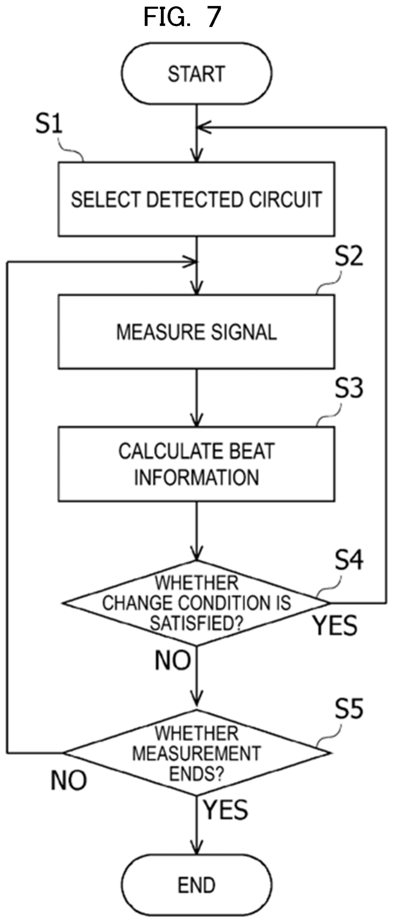

FIG. 7 is a diagram illustrating an example of a processing flow of the control body 12 according to the embodiment. Hereinafter, an example of a processing flow of the control body 12 according to the embodiment will be described with reference to FIG. 7.

In step S1, the selection unit 21 selects, from the detection circuits 14 included in the electrocardiograph 1, a supplied circuit to which power is supplied from the battery 121. The selection of the supplied circuit is, for example, as described with reference to FIG. 6A to FIG. 6D. When processing of step S1 is executed at the time of activation of the electrocardiograph 1, the selection unit 21 may receive designation of the electrodes 11 by the user, and the detection circuit 14 corresponding to the designated electrodes 11 may be determined to be the supplied circuit.

In step S2, the measurement unit 22 acquires an electrocardiographic signal by using the detection circuit 14 selected in step S1 and the electrodes 11 connected to the detection circuit 14. In step S3, the calculation unit 23 calculates beat information based on the electrocardiographic signal acquired in step S3. Examples of the beat information can include a beat rate, an RRI, and the like.

In step S4, the selection unit 21 determines whether the beat information calculated in step S3 satisfies a change condition for changing the detection circuit 14 to which power is to be supplied. Examples of the change condition can include a case where a symptom of a heart disease such as atrial fibrillation is detected, a case where noise included in the electrocardiographic signal acquired by the measurement unit 22 is equal to or greater than the threshold value, a case where it is determined based on the electrocardiographic signal that there is no heart abnormality, a case where the RRI of the electrocardiographic signal is irregular, and the like. When the change condition is satisfied (YES in step S4), the processing proceeds to step S1. When the change condition is not satisfied (NO in step S4), the processing proceeds to step S5.

In step S5, whether to end the measurement is determined. Examples of whether to end the measurement can include a case where a predetermined measurement time has ended and a case where an instruction to end the measurement is received from the user. When the measurement ends (YES in step S5), the processing ends. When the measurement does not end (NO in step S5), the processing proceeds to step S2.

### <Advantageous Effects of Embodiments>

When a large number of the electrodes 11 and the detection circuits 14 to which the electrodes 11 are connected are arranged on the band 10 and power is supplied from the battery 121 to all the detection circuits 14, the power consumption of the electrocardiograph 1 increases, and thus the time during which the electrocardiograph 1 can continue the electrocardiographic measurement is likely to decrease. On the other hand, when the minimum number of the electrodes 11 (for example, two electrodes 11) and the detection circuit 14 to which the electrodes 11 are connected are arranged on the band 10 to perform electrocardiographic measurement, the power consumption of the electrocardiograph 1 can be reduced, but whether the arranged two electrodes 11 are at positions suitable for the electrocardiographic measurement becomes a problem. In the present embodiment, by arranging a large number (six) of the electrodes 11 on the band 10, any of the electrodes 11 is expected to be disposed at a position suitable for the electrocardiographic measurement. Additionally, in the present embodiment, the power from the battery 121 is not always supplied to all the detection circuits 14, but the power from the battery 121 is supplied to the detection circuit 14 selected by the selection unit 21. Therefore, according to the present embodiment, the power consumption of the electrocardiograph 1 can be reduced, and the measurement time of the electrocardiographic measurement by the electrocardiograph 1 can be increased as long as possible.

In the present embodiment, when the noise included in the electrocardiographic signal acquired by some of the electrodes 11 becomes equal to or greater than a predetermined noise threshold value, the acquisition of electrocardiographic signals by all the electrodes 11 arranged on the band 10 is started. The electrode 11 other than the some electrodes 11 may include an electrode 11 that can acquire an electrocardiographic signal with less noise. Therefore, according to the present embodiment, even when the noise is included in the electrocardiographic signal acquired by any of the electrodes 11, the electrocardiographic measurement can be continued.

In the present embodiment, when a deviation of the RRI is equal to or greater than the predetermined threshold value, the acquisition of electrocardiographic signals by all the electrodes 11 and the detection circuits 14 that are arranged on the band 10 is started. When the deviation of the RRI is equal to or greater than the threshold value, the occurrence of a heart disease such as atrial fibrillation is suspected. In the present embodiment, when a heart disease is suspected, the acquisition of electrocardiographic signals by all the electrodes 11 and the detection circuits 14 is started, and thus more detailed electrocardiographic measurement can be performed.

In the present embodiment, power is supplied to all the detection circuits 14 for a predetermined period, and the detection circuit 14 to which power is supplied from the battery 121 is selected based on the RRI of the electrocardiographic signal acquired in the predetermined period. Therefore, according to the present embodiment, the electrocardiographic signals acquired for all the plurality of electrodes 11 and the detection circuits 14 are compared, and the supply of power by the battery 121 can be performed on the detection circuit 14 more suitable for acquiring the electrocardiographic signal.

In addition, in the present embodiment, since the electrodes 11 are disposed on the band 10, it is easy to wear the electrocardiograph 1 so that the electrodes 11 are in contact with the living body of the subject.

### <First Modified Example>

In the embodiment described above, the detection circuits 141, 142, 143, 144, and 145 to which pairs of the electrode 116, the potential of which is fixed to the ground and the other electrode 11 are connected are provided. However, the connection between the electrode 11 and the detection circuit 14 is not limited to such a configuration. In a first modified example, variations of the connection between the electrode 11 and the detection circuit 14 will be described.

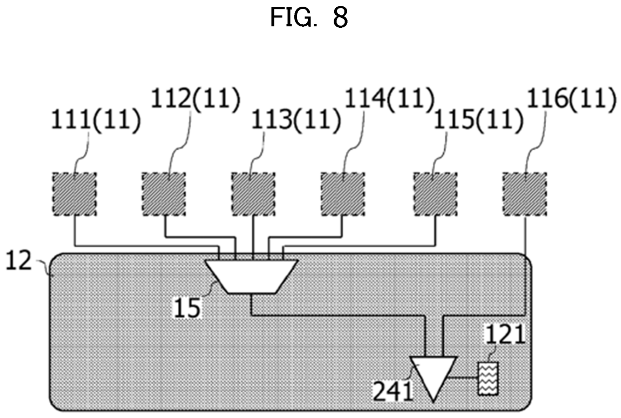

FIG. 8 to FIG. 10 are diagrams illustrating variations of the connection between the electrode 11 and the detection circuit 14 in the first modified example. In FIG. 8, the electrodes 111, 112, 113, 114, and 115 are connected to a switch 15. The output of the switch is connected to a detection circuit 241. The electrode 116, the potential of which is fixed to the ground is connected to the detection circuit 241. The switch 15 selects, from among the electrodes 111, 112, 113, 114, and 115, the electrode 11 to be connected to the detection circuit 241, and switches to the selected electrode 11. The switch 15 is switched by, for example, the selection unit 21. The configuration illustrated in FIG. 8 also enables switching of the electrodes 11 used for acquiring an electrocardiographic signal. In addition, the power consumption of the detection circuit 241 can be reduced more in a case where the selected some electrodes 11 are used than in a case where all the electrodes 11 are used.

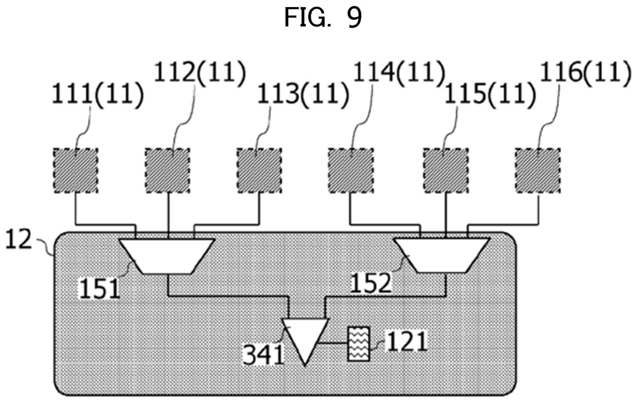

In FIG. 9, two switches 151 and 152 are prepared as switches for switching the electrodes 11. The electrodes 111, 112, and 113 are connected to the switch 151. The electrodes 114, 115, and 116 are connected to the switch 152. The outputs of the switches 151 and 152 are input to a detection circuit 141. The switch 151 switches, from among the electrodes 111, 112, and 113, the electrode 11 to be connected to the detection circuit 141. The switch 152 switches, from among the electrodes 114, 115, and 116, the electrode 11 to be connected to the detection circuit 141. The switches 151 and 152 are switched by, for example, the selection unit 21. In the configuration of FIG. 9, the potential of the electrode 116 is not fixed to the ground. The configuration illustrated in FIG. 9 also enables switching of the electrodes 11 used for acquiring an electrocardiographic signal. In addition, the power consumption of the detection circuit 341 can be reduced more in a case where the selected some electrodes 11 are used than in a case where all the electrodes 11 are used.

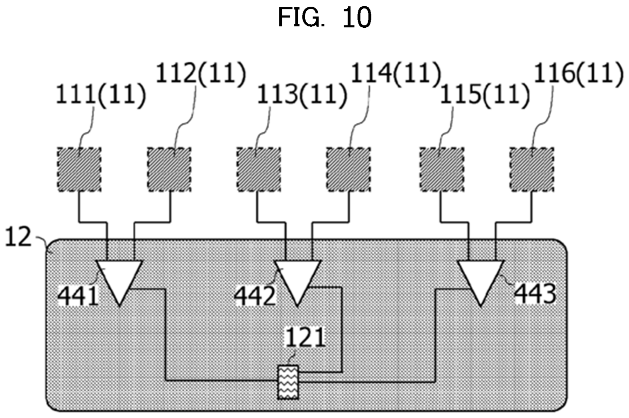

In FIG. 10, detection circuits 441, 442, and 443, to each of which a pair of the electrodes 11 is connected are prepared. The electrodes 111 and 112 are connected to the detection circuit 441. The electrodes 113 and 114 are connected to the detection circuit 442. The electrodes 115 and 116 are connected to the detection circuit 443. The selection unit 21 selects, from among the detection circuits 441, 442, and 443, for example, a detection circuit to which power from the battery 121 is supplied. In the configuration of FIG. 10, the potential of the electrode 116 is not fixed to the ground. The configuration illustrated in FIG. 10 also enables switching of the electrodes 11 used for acquiring an electrocardiographic signal. In addition, the power consumption of the electrocardiograph 1 can be reduced more in the configuration illustrated in FIG. 10 in which the electrocardiographic signal is measured using some of the detection circuits than the configuration in which the electrocardiographic signals are measured using all the detection circuits 441, 442, and 443.

### <Second Modified Example>

In the embodiment described above, the electrocardiographic signal is acquired using the selected some the electrodes 11, and the number of the electrodes 11 used for acquiring electrocardiographic signals is increased when a symptom of a heart disease is detected, when noise increases, or the like. In a second modified example, a configuration in which a pair of the electrodes 11 used for acquiring electrocardiographic signals is switched within a sampling period will be described.

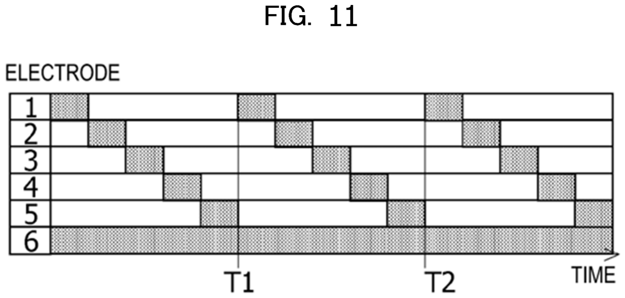

FIG. 11 is a diagram schematically illustrating selection of the electrodes 11 used for acquiring an electrocardiographic signal in the second modified example. In FIG. 11, similarly to FIG. 6, the electrode 11 with the number "1" corresponds to the electrode 111, the electrode 11 with the number "2" corresponds to the electrode 112, the electrode 11 with the number "3" corresponds to the electrode 113, the electrode 11 with the number "4" corresponds to the electrode 114, the electrode 11 with the number "5" corresponds to the electrode 115, and the electrode 11 with the number "6" corresponds to the electrode 116. In the example of FIG. 11, the electrode 116 with the number "6" is a GND electrode, the potential of which is fixed to the ground.

The selection unit 21 switches the electrode 11 to be combined with the electrode 116, the potential of which is set to the ground within the sampling period. The switching of the electrodes 11 may be realized by, for example, as described in FIG. 8, connecting the electrodes 111, 112, 113, 114, and 115 to the switch 15 and switching, by the selection unit 21, which of the electrodes 111, 112, 113, 114, and 115 is connected to the detection circuit 241. FIG. 11 illustrates an example in which the electrodes 111, 112, 113, 114, and 115 are connected to the detection circuit 241 in this order within the sampling period. In FIG. 11, the sampling period is exemplified by T1 and T2, for example. According to the second modified example, since the electrocardiographic signal is acquired using some of the electrodes 11 within the sampling period, the power consumption of the electrocardiograph 1 can be reduced.

### <Other Modified Examples>

In the embodiment described above, when a deviation of the RRI is equal to or larger than the threshold value, the acquisition of electrocardiographic signals by all the electrodes 11 arranged on the band 10 is started. However, the start of acquisition of electrocardiographic signals by all the electrodes 11 may be triggered by another event. For example, when no P wave is detected in the electrocardiographic signal acquired by the some electrodes 11, the selection unit 21 may start the acquisition of electrocardiographic signals by all the electrodes 11.

In the embodiment described above, when the deviation of the RRI is equal to or larger than the threshold value, the acquisition of electrocardiographic signals by all the electrodes 11 arranged on the band 10 and the detection circuits 14 is started, but the electrocardiograph 1 is not limited to such an aspect. For example, when the deviation of the RRI is equal to or greater than the threshold value, the electrocardiograph 1 may acquire the electrocardiographic signal using more detection circuits 14 than the selected detection circuits 14.

The embodiments and modified examples disclosed above can be combined.

### <Supplementary Note 1>

A biosignal measurement device (1), including:
a plurality of electrodes (11);
a measurement unit (22) including a detection circuit (14) that detects a potential difference using the plurality of electrodes (11) and configured to measure an electrocardiographic signal of a subject using the detection circuit (14);
a battery (121) configured to supply power to the detection circuit (14); and
a selection unit (21) configured to select a measurement electrode (11) to be used for measurement of the electrocardiographic signal of the subject from the plurality of electrodes (11), based on the electrocardiographic signal of the subject, which is measured using at least some of the plurality of electrodes (11) and the detection circuit (14),
in which the measurement unit (22) measures the electrocardiographic signal using the detection circuit and the measurement electrode.

### <Supplementary Note 2>

The biosignal measurement device according to Supplementary Note 1, in which when an abnormality is detected in the electrocardiographic signal measured using the at least some of the plurality of electrodes (11), the selection unit (21) increases the number of the electrodes (11) to be selected as the measurement electrodes.

### <Supplementary Note 3>

The biosignal measurement device (1) according to Supplementary Note 1, in which when an abnormality is detected in the electrocardiographic signal measured using the at least some of the plurality of electrodes (11), the selection unit (21) selects all the plurality of electrodes (11) as the measurement electrodes (11).

### <Supplementary Note 4>

The biosignal measurement device (1) according to Supplementary Note 2 or 3, in which the abnormality of the electrocardiographic signal includes a case where a deviation of an R-R Interval (RRI) in a time-series change of the electrocardiographic signal is equal to or greater than a first threshold value.

### <Supplementary Note 5>

The biosignal measurement device (1) according to any one of Supplementary Notes 2 to 4, in which the abnormality of the electrocardiographic signal includes a case where a P wave is not detected in the electrocardiographic signal.

### <Supplementary Note 6>

The biosignal measurement device (1) according to any one of Supplementary Notes 2 to 5, in which the abnormality of the electrocardiographic signal includes a case where a magnitude of noise included in the electrocardiographic signal is equal to or greater than a second threshold value.

### <Supplementary Note 7>

The biosignal measurement device (1) according to any one of Supplementary Notes 1 to 6, in which the selection unit (21) allows the measurement of the electrocardiographic signal using all the plurality of electrodes (11) to be executed at predetermined intervals, and determines, based on the electrocardiographic signal measured using all the plurality of electrodes (11), which electrode (11) of the plurality of electrodes (11) is to be the measurement electrode (11).

### <Supplementary Note 8>

The biosignal measurement device (1) according to any one of Supplementary Notes 1 to 7, further including a fixing member having a belt-like configuration to be wound on the subject,
in which the plurality of electrodes are disposed on the fixing member.

### REFERENCE SIGNS

1 Electrocardiograph
10 Band
11 Electrode
12 Control body
13 Fixing mechanism
14 Detection circuit
15 Switch
21 Selection unit
22 Measurement unit
23 Calculation unit
24 Storage unit
25 Output unit
111 Electrode
112 Electrode
113 Electrode
114 Electrode
115 Electrode
116 Electrode
141 Detection circuit
142 Detection circuit
143 Detection circuit
144 Detection circuit
145 Detection circuit
151 Switch
152 Switch
121 Battery
241 Detection circuit
341 Detection circuit
441 Detection circuit
442 Detection circuit
443 Detection circuit

## Claims

1. A biosignal measurement device, comprising:
a plurality of electrodes;
a measurement unit including a detection circuit that detects a potential difference using the plurality of electrodes and configured to measure an electrocardiographic signal of a subject using the detection circuit;
a battery configured to supply power to the detection circuit; and
a selection unit configured to select a measurement electrode to be used for measurement of the electrocardiographic signal of the subject from the plurality of electrodes, based on the electrocardiographic signal of the subject, which is measured using at least some of the plurality of electrodes and the detection circuit,
wherein the measurement unit measures the electrocardiographic signal using the detection circuit and the measurement electrode.

2. The biosignal measurement device according to claim 1, wherein when an abnormality is detected in the electrocardiographic signal measured using the at least some of the plurality of electrodes, the selection unit increases the number of the electrodes to be selected as the measurement electrodes.

3. The biosignal measurement device according to claim 1, wherein when an abnormality is detected in the electrocardiographic signal measured using the at least some of the plurality of electrodes, the selection unit selects all the plurality of electrodes as the measurement electrodes.

4. The biosignal measurement device according to claim 2, wherein the abnormality of the electrocardiographic signal includes a case where a deviation of an R-R Interval (RRI) in a time-series change of the electrocardiographic signal is equal to or greater than a first threshold value.

5. The biosignal measurement device according to claim 2, wherein the abnormality of the electrocardiographic signal includes a case where a P wave is not detected in the electrocardiographic signal.

6. The biosignal measurement device according to claim 2, wherein the abnormality of the electrocardiographic signal includes a case where a magnitude of noise included in the electrocardiographic signal is equal to or greater than a second threshold value.

7. The biosignal measurement device according to claim 1, wherein the selection unit allows the measurement of the electrocardiographic signal using all the plurality of electrodes to be executed at predetermined intervals, and determines, based on the electrocardiographic signal measured using all the plurality of electrodes, which electrode of the plurality of electrodes is to be the measurement electrode.

8. The biosignal measurement device according to any one of claims 1 to 7, further comprising a fixing member having a belt-like configuration to be wound on the subject,
wherein the plurality of electrodes are disposed on the fixing member.
